# EUROPEAN PATENT APPLICATION

(11) **EP 2 510 871 A1**
(43) Date of publication of application: **17.10.2012**
(21) Application number: 12159033.5
(22) Date of filing: 12.03.2012
(51) Int. Cl.: A61B 5/04

(54) **Wearing structure for measuring physiological signal**

(30) Priority: 13.04.2011 TW 100112779
(71) Applicant: King's Metal Fiber Technologies Co., Ltd., Taipei City (TW)
(72) Inventor: Hsiao, King-Mu, Taipei City (TW); Huang, Hong-Hsu, Taipei City (TW); Su, I-Chen, Taipei City (TW); Peng, Jung-Hsiang, Taipei City (TW)
(74) Representative: von Kreisler Selting Werner

(57) **Abstract**

A wearing structure for measuring a physiological signal is provided. The wearing structure includes a sensing layer, a liquid storing layer and a cover layer. The sensing layer has at least one sensor for sensing the physiological signal of a user. The liquid storing layer covers the sensing layer and stores an electrically conductive liquid. The cover layer covers the storing layer for preventing loss of the electrically conductive liquid. The liquid storing layer is disposed between the sensing layer and the cover layer. The electrically conductive liquid is used for enhancing the sensing of the physiological signal of the user by the at least one sensor.

## Description

This application claims priority to Taiwan Patent Application No. 100112779 filed on April 13, 2011, which is hereby incorporated by reference in its entirety.

### CROSS-REFERENCES TO RELATED APPLICATIONS

Not applicable.

### BACKGROUND OF THE INVENTION

### Field of the Invention

The present invention relates to a wearing structure for measuring a physiological signal. In particular, the wearing structure of the present invention utilizes an electrically conductive liquid stored therein to enhance the sensing of the physiological signal by a sensor woven from metal fibers.

### Descriptions of the Related Art

Observing the reactions of a human body under various conditions is very important for improving the quality of people's life. The evaluation of human body conditions helps to further improve one's health or to motivate one's potential power. For example, in the medical field, by periodically measuring or evaluating the physiological status of a patient, the physical changes of the patient can be known in real time so that the most appropriate medical treatment and care can be given. In the sports field, by measuring the physiological statuses of sportsmen in a training course or a game, the optimal training mode can be determined according to the respective training loads of the different sportsmen so that their potentials can be reached. As the science and technology continually advances, the method in which the physiological status can be accurately sensed has become an important issue.

Currently, the measurement of a user's physiological status (e.g., a myoelectric status or an electrocardiac status) is mostly accomplished by placing a transmission medium connected with a monitor onto a particular site of the user's body. Then, the physiological signal of the user's body is sensed by the transmission medium and then displayed on the monitor in the form of a graph, from which the user's physiological status can be known. Generally, the transmission medium commonly used for sensing is an electrode patch or a fabric with electrodes.

In more detail, the electrode patch is generally used in the following way: electrodes on the patch are attached to the user's skin tightly by means of an adhesive material of the patch, and then the physiological signal of the user is measured by the electrodes and transmitted to the monitor so that the current physiological status of the user is determined. Similarly, the fabric with electrodes is worn by or fixed to the user in such a way that the electrodes in the fabric are in tight contact with the user's skin, and then a physiological signal is measured by the electrodes and transmitted to the monitor so that the current physiological status of the user is determined.

However, because the adhesive material of the electrode patch tends to lose its adhesion after repeated use, it becomes difficult for the electrode patch to be adhered to the user's skin, which may adversely affect the sensing result of the user's physiological status. On the other hand, the fabric with electrodes is only loosely coming into contact with the user since it is worn, so an undue gap usually exists between the fabric and the user's skin during normal use and this also leads to a weak sensing signal.

Accordingly, an urgent need exists in the art to solve the aforesaid problems with the prior art so as to improve the accuracy of sensing a physiological signal.

### SUMMARY OF THE INVENTION

To obviate the inconveniences in using an electrode patch or a fabric with electrodes as a transmission medium to measure a user's physiological signal, the present invention provides a wearing structure for measuring a physiological signal. The wearing structure of the present invention utilizes an electrically conductive liquid to enhance the sensing of the user's physiological signal by a sensor in the wearing structure, so the user's physiological status can be known more accurately.

The wearing structure of the present invention is reflected in independent claim 1.

### Preferred embodiments are indicated in the dependent claims.

To achieve the aforesaid objective, the wearing structure for measuring a physiological signal of the present invention comprises a sensing layer, a liquid storing layer and a cover layer. The sensing layer has at least one sensor for sensing the physiological signal of a user. The liquid storing layer covers the sensing layer and stores an electrically conductive liquid. The cover layer covers the liquid storing layer for preventing loss of the electrically conductive liquid. The liquid storing layer is disposed between the sensing layer and the cover layer, while the electrically conductive liquid is used for enhancing the sensing of the physiological signal of the user by the at least one sensor.

With the technical features disclosed above, the wearing structure of the present invention can utilize the electrically conductive liquid to further enhance the sensing of the physiological signal so that the physiological signal can be measured more conveniently and more accurately. Furthermore, the wearing structure of the present invention may be formed as various wearable articles that can be conveniently worn by the user at sites to be sensed of the user's body.

The detailed technology and preferred embodiments implemented for the subject invention are described in the following paragraphs accompanying the appended drawings for people skilled in this field to well appreciate the features of the claimed invention.

### BRIEF DESCRIPTION OF THE DRAWINGS

**FIG. 1** is a cross-sectional view of a wearing structure according to a first embodiment of the present invention;
**FIGS. 2A** and **2B** are cross-sectional views of a wearing structure according to a second embodiment of the present invention;
**FIG 3** is a cross-sectional view of a wearing structure according to a third embodiment of the present invention; and
**FIG. 4** is a cross-sectional view of a wearing structure according to a fourth embodiment of the present invention.

### DESCRIPTION OF THE PREFERRED EMBODIMENT

In the following descriptions, the present invention will be explained with reference to embodiments thereof. However, these embodiments are not intended to limit the present invention to any specific environment, applications or particular implementations described in these embodiments. Therefore, the descriptions of these embodiments are only for purposes of illustration but not to limit the present invention. It should be appreciated that in the following embodiments and the attached drawings, elements unrelated to the present invention are omitted from depiction.

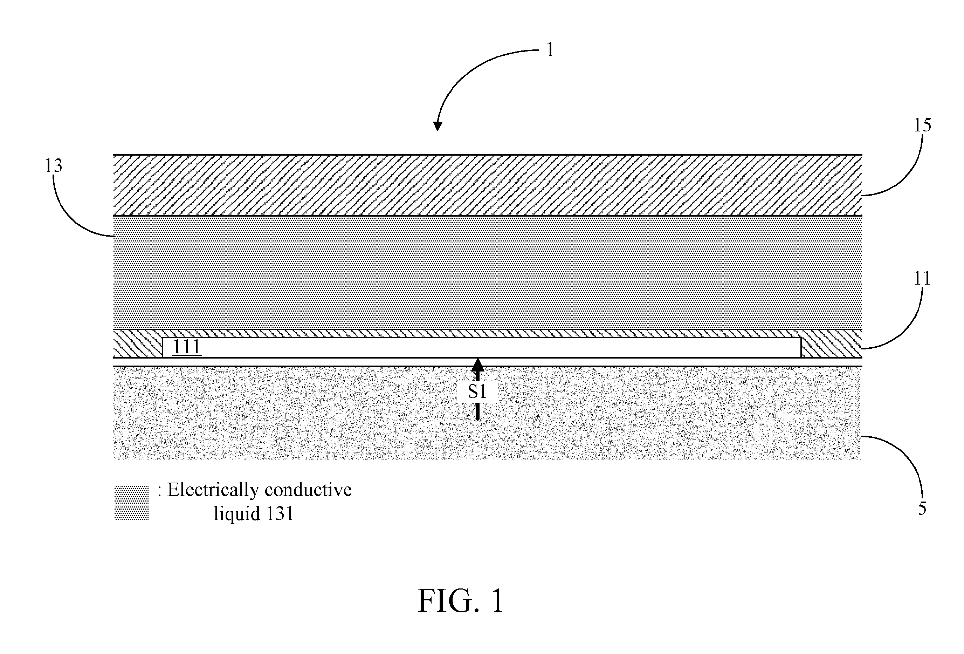

Firstly, referring to **FIG. 1** there is shown a cross-sectional view of a wearing structure **1** according to a first embodiment of the present invention. The wearing structure **1** comprises a sensing layer **11,** a liquid storing layer **13** and a cover layer **15.** As shown, the liquid storing layer **13** covers the sensing layer **11** and, in turn, the cover layer **15** covers the liquid storing layer **13;** i.e., the liquid storing layer **13** is disposed between the sensing layer **11** and the cover layer **15.** The functions of the individual elements of the wearing structure **1** will be detailed hereinbelow.

Specifically, the sensing layer **11** of the wearing structure **1** comes into direct contact with a user **5.** The sensing layer **11** comprises at least one sensor **111** for sensing a physiological signal **S1** of the user **5.** The sensing layer **11** is made of a metal, such as stainless steel fibers, copper fibers, carbon fibers or sputtered silver, via weaving, in the form of a fabric. The liquid storing layer **13** is used to store an electrically conductive liquid **131** for increasing the humidity of the wearing structure **1** so that the clearance between the user **5** and the at least one sensor **111** can be filled by the electrically conductive liquid **131.** This can improve the electrical conduction capability of the at least one sensor **111** and further enhance the sensing of the physiological signal **S1** of the user 5 so that the physiological signal **S1** can be subsequently transmitted to a monitor (not shown) communicatively connected to the at least one sensor **111.** It shall be particularly appreciated that the electrically conductive liquid **131** in the first embodiment may be a common electrically conductive liquid, such as impure water (because pure water is nonconductive) including tap water, although this is not intended to limit implementations of the electrically conductive liquid.

However, when the electrically conductive liquid **131** is stored in the liquid storing layer **13,** it is possible that the electrically conductive liquid **131** might be loosened from the other side of the liquid storing layer **13** opposite to the side that comes into contact with the user **5.** For this reason, a cover layer **15** is additionally provided to prevent loss of the electrically conductive liquid **131.** Hence, the cover layer **15** may be made of a water-proof material impermeable by the air. Thus, loss of the electrically conductive liquid **131** stored in the liquid storing layer **13** can be prevented by the cover layer **15** covering the liquid storing layer **13**.

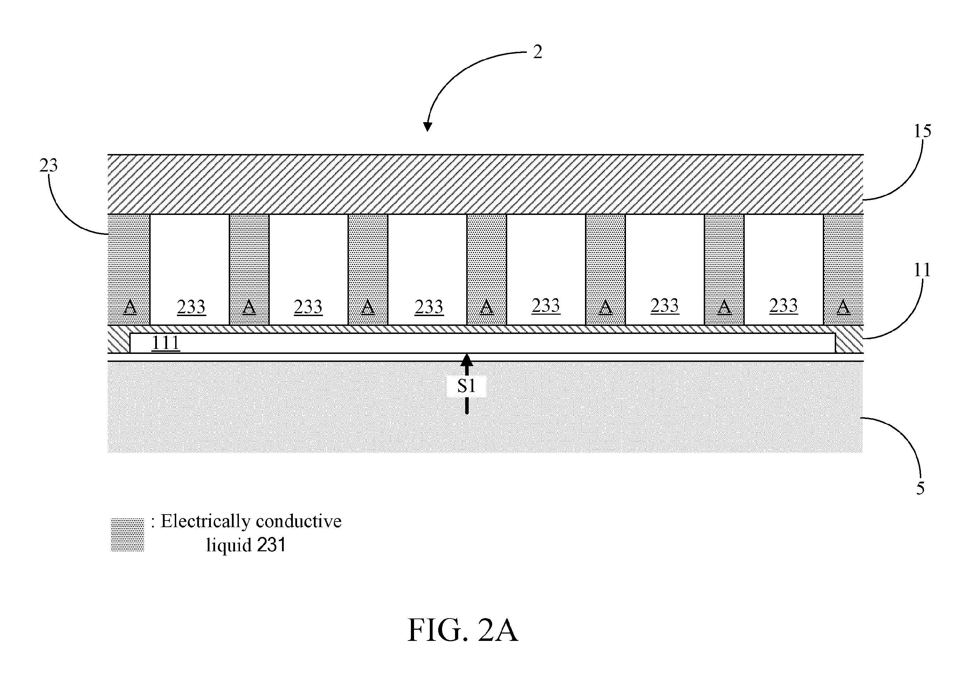

Next, referring to **FIG.** 2A which illustrates a cross-sectional view of a wearing structure **2** according to a second embodiment of the present invention. The wearing structure **2** of the second embodiment is substantially the same as the wearing structure **1** of the first embodiment, so elements bearing the same reference numerals and functions thereof will not be described again herein. The second embodiment differs from the first embodiment in the liquid storing layer.

Specifically, the wearing structure **2** of the second embodiment has a liquid storing layer **23** different from that of the first embodiment. In more detail, the liquid storing layer **23** stores an electrically conductive liquid **231** for increasing the humidity of the wearing structure **2.** However, the liquid storing layer **23** comprises a plurality of filling elements **233** that define a plurality of storing spaces A in the liquid storing layer **23** for storing the electrically conductive liquid **231.**

Further speaking, in the second embodiment, each of the filling elements **233** is a solid-state column, for example, a small-size column made of nylon, carbon fibers, polyethylene (PE), polypropylene (PP) or polyvinylidene fluoride (PVDF). By arranging the filling elements **233** regularly, the filling elements **233** support the sensing layer **21** and the cover layer **25**;meanwhile, make the wearing structure **2** flexible so that it can be attached to any site of the user's body conveniently.

As shown, the filling elements **233** define a plurality of storing spaces A in the liquid storing layer **23** as shown. When intervals between the filling elements **233** are relatively small, the adhesion between the electrically conductive liquid **231** and the filling elements **233** is greater than a cohesion force of the electrically conductive liquid **231** itself and the so-called capillary phenomenon results. As a result, the electrically conductive liquid **231** can be easily stored in the liquid storing layer **23** by being absorbed onto the filling elements **233**.

More specifically, when a physiological signal **S1** of the user needs to be sensed, the electrically conductive liquid **231** is sprayed onto the wearing structure **2.** Because of the aforesaid capillary phenomenon, the electrically conductive liquid **231** is absorbed into the storing spaces A defined between the filling elements **233.** Thereby, the electrically conductive liquid 231 are stored in the wearing structure **2.** Alternatively, the sweat of the user may be used directly as the electrically conductive liquid **231;** in other words, when the user 5 sweats, the sweat will be absorbed into the liquid storing layer **23** due to the capillary phenomenon and be stored in the storing spaces A defined by the filling elements **233.**

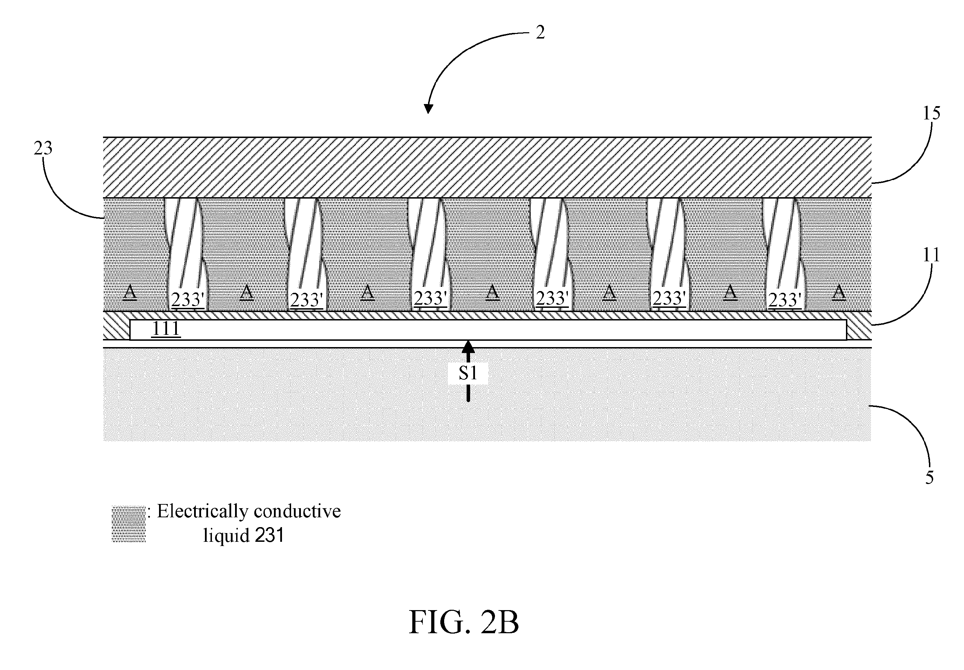

It shall be particularly appreciated that each of the filling elements **233** is formed as a single-core structure, but this is not intended to limit implementations of the filling elements **233.** Referring to **FIG. 2B****,** in other examples of the second embodiment, the filling elements of the liquid storing layer **23** may also be implemented as filling elements **233'** of a multi-core structure. As shown in **FIG. 2B****,** each of the filling elements **233'** is formed by a plurality of small-size strip-like elements twisted together. This multi-core structure can further enhance the liquid storing function of the liquid storing layer **23.**

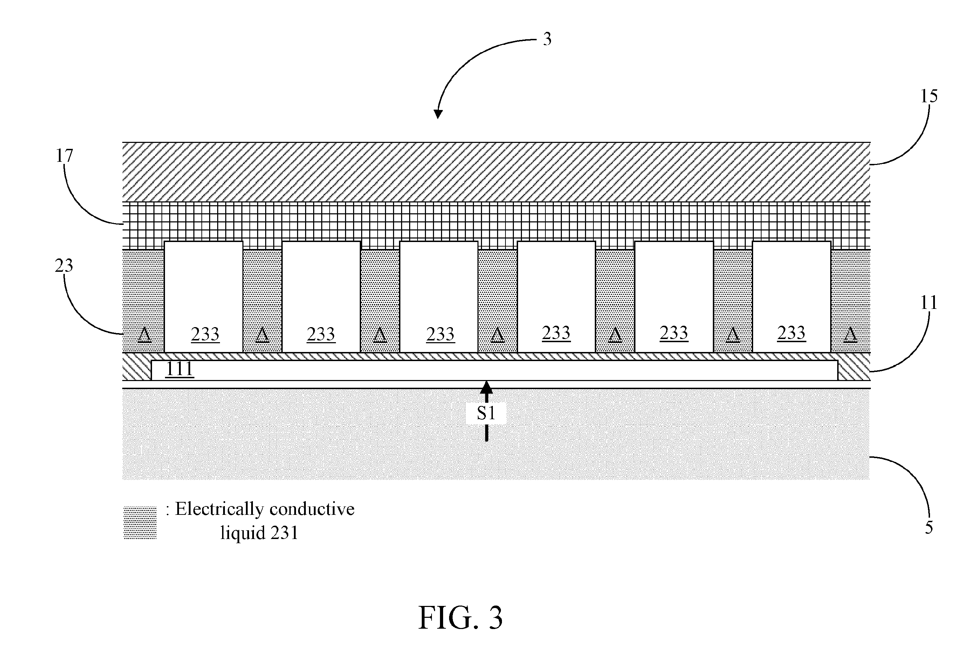

Next, referring to **FIG. 3** there is shown a cross-sectional view of a wearing structure **3** according to a third embodiment of the present invention. The wearing structure **3** of the third embodiment is substantially the same as the wearing structure **2** of the second embodiment, so elements bearing the same reference numerals and functions thereof will not be described again herein. The third embodiment differs from the second embodiment in that the wearing structure **3** of the third embodiment is additionally provided with a fixing layer **17.**

Specifically, the fixing layer **17** is disposed between the liquid storing layer **23** and the cover layer **15** to fix the filling elements **233** in place in the liquid storing layer **23.** It shall be particularly emphasized that the fixing layer **17** may be made of an elastic fabric, although this is not intended to limit the material of the fixing layer **17.**

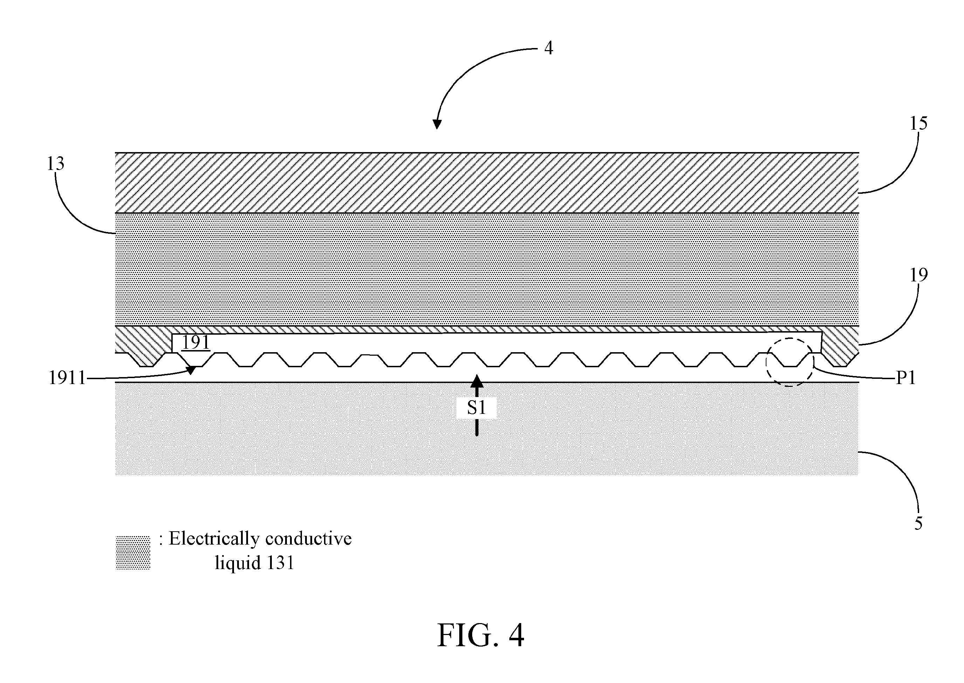

Referring to **FIG. 4****,** there is shown a cross-sectional view of a wearing structure **4** according to a fourth embodiment of the present invention. The wearing structure **4** of the fourth embodiment is substantially the same as the wearing structure **1** of the first embodiment, so elements bearing the same reference numerals and functions thereof will not be described again herein. The fourth embodiment differs from the first embodiment in the sensing layer.

Specifically, in order for the wearing structure **4** to come into closer contact with the user **5** so that the sensor can measure the user's physiological signal more accurately, a sensing surface **1911** of at least one sensor **191** of a sensing layer **19** has a plurality of protrusions **P1** in the fourth embodiment.

More specifically, the sensing surface **1911,** which comes into contact with the user's body, of the at least one sensor **191** of the sensing layer **19** has a plurality of protrusions **P1,** as shown in **FIG. 4****.** In use, this structure can remarkably enhance the tightness of the contact between the user 5 and the sensing surface **1911,** which can further enhance the sensing of the physiological signal **S1** of the user **5.** It shall be particularly appreciated that the protrusions **P1** are rounded protrusions in the fourth embodiment so that the user **5** will not feel uncomfortable when the wearing structure **4** comes into contact with the user's body; however, this is not intended to limit implementations of the protrusions **P1.**

According to the above descriptions, the wearing structure of the present invention allows the sensor thereof to sense a user's physiological signal accurately. Thereby, the disadvantages of the conventional practices of using an electrode patch or a common fabric with electrodes as a sensing medium are easily overcome; as a result, the sensing of the user's physiological signal can be accomplished more efficiently. Furthermore, the wearing structure of the present invention may be formed as various wearable articles that can be conveniently worn by the user at sites to be sensed of the user's body.

The above disclosure is related to the detailed technical contents and inventive features thereof. People skilled in this field may proceed with a variety of modifications and replacements based on the disclosures and suggestions of the invention as described without departing from the characteristics thereof. Nevertheless, although such modifications and replacements are not fully disclosed in the above descriptions, they have substantially been covered in the following claims as appended.

## Claims

1. A wearing structure for measuring a physiological signal, comprising:
a sensing layer, having at least one sensor for sensing the physiological signal of a user;
a liquid storing layer, covering the sensing layer and storing an electrically conductive liquid; and
a cover layer, covering the liquid storing layer for preventing the electrically conductive liquid from losing;
wherein the liquid storing layer is disposed between the sensing layer and the cover layer, and the electrically conductive liquid is used for enhancing the sensing of the physiological signal of the user by the at least one sensor.

2. The wearing structure as claimed in claim 1, wherein the liquid storing layer comprises a plurality of filling elements, and the filling elements define a plurality of storing spaces in the liquid storing layer for storing the electrically conductive liquid.

3. The wearing structure as claimed in claim 2, further comprising a fixing layer which is disposed between the cover layer and the liquid storing layer for fixing the filling elements.

4. The wearing structure as claimed in claim 2 or 3, wherein the filling elements are made of a material selected from the group consisting of nylon, carbon fibers, polyethylene (PE), polypropylene (PP) or polyvinylidene fluoride (PVDF).

5. The wearing structure as claimed in one or more of claims 2 to 4, wherein each of the filling elements is formed as a single-core structure.

6. The wearing structure as claimed in one or more of claims 2 to 4, wherein each of the filling elements is formed as a multi-core structure.

7. The wearing structure as claimed in one or more of claims 1 to 6, wherein the sensing layer is made of a material selected from the group consisting of metal, stainless steel fibers, copper fibers, carbon fibers or sputtered silver.

8. The wearing structure as claimed in one or more of claims 1 to 6, wherein a sensing surface of the at least one sensor has a plurality of protrusions.
